# EUROPEAN PATENT SPECIFICATION

(11) **EP 3 762 059 B1**
(45) Date of publication and mention of the grant of the patent: **21.08.2024**
(21) Application number: 19709230.7
(22) Date of filing: 19.02.2019
(51) Int. Cl.: A61M 1/14, A47K 1/14, A61M 1/28, F16L 3/00

(54) **DEVICES FOR SECURING DIALYSIS FLUID LINES AND RELATED SYSTEMS**
VORRICHTUNGEN ZUR FIXIERUNG VON DIALYSEFLÜSSIGKEITSLEITUNGEN SOWIE ZUGEHÖRIGE SYSTEME
DISPOSITIFS DE FIXATION DE LIGNES DE FLUIDE DE DIALYSE ET SYSTÈMES

(30) Priority: 08.03.2018 US 201815915586
(43) Date of publication of application: 13.01.2021
(73) Proprietor: Fresenius Medical Care Holdings, Inc., Waltham, MA 02451-1457 (US)
(72) Inventor: SUAREZ DEL REAL PENA, Diego, Mission, Texas 78572 (US); HERNANDEZ, Irving, Rio Bravo, 88920 (MX)
(74) Representative: Fish & Richardson P.C.
(86) International application number: PCT/US2019/018558
(87) International publication number: WO 2019/173043

(56) References cited:
- GB-A- 2 448 863
- US-A- 4 722 556
- US-A- 5 096 233
- US-A- 5 201 553
- US-A1- 2013 345 622
- US-A1- 2017 281 846
- US-B1- 8 382 047
- US-B2- 7 090 180

## Description

### TECHNICAL FIELD

This specification relates to devices for securing dialysis fluid lines, and related systems and methods for securing dialysis fluid lines.

### BACKGROUND

Extracorporeal treatments such as hemodialysis and peritoneal dialysis (hereinafter referred to as "PD") treatments can be used to filter waste products from a patient's bloodstream. For example, in a hemodialysis treatment, a hemodialysis machine can pump dialysate and the patient's blood alongside one another through a dialyzer. The waste products in the patient's blood can be drawn into the dialysate. The dialysate containing the waste products can then be sent to a drain bag or drain for disposal. In a PD treatment, a PD machine can pump fresh dialysate from dialysate containers into the peritoneum of a patient. The dialysate draws waste products from the patient's bloodstream. The dialysate fluid containing the waste products can then be sent to a drain bag or drain for disposal.

GB 2448863 describes a bath plug that comprises a tapered circular plug base member to fit a bath drain hole, a tube with an inlet opening at one end and adapted to receive a hose pipe at the other end of the tube, and means to support the tube on the plug base member.

US 8,382,047 describes a multi-use support or clamping device for supporting at least one hose is mountable to a fixture, such as a sink, toilet, or other waste receptacle, for facilitating discharge of a liquid waste directly into the waste receptacle. The multi-use clamping device includes a clamp, a hose support, an attachment mechanism, and an anchoring mechanism. The clamp is removably securable to the waste receptacle or other fixture. The hose support is an elongated cylindrical member having a bore therethrough sized for disposition of at least one hose, such as a dialysis hose, a cable, tubing, or any other elongated piece of material.

US 2013/0345622 describes a dialysis system includes a source of effluent dialysis fluid, a drain container configured to receive the effluent dialysis fluid through a drain tube, and a load cell. The drain container includes an at least semi-rigid body defining a first key feature. The load cell includes a second, mating key feature positioned and arranged to mate with the first key feature.

US 2017/0281846 describes systems for generating peritoneal dialysate that use a water purification module, a sterilization module, and concentrates to prepare peritoneal dialysate from source water and infuse the prepared peritoneal dialysate into a patient with an integrated cycler.

US 7,090,179 describes a mounting assembly for a discharge conduit of a medical treatment device, such as a dialysis machine. The mounting assembly has a connector member detachably engageable with a discharge nozzle of a discharge conduit and a suction cup that can be mounted on a rim of a waste receptor, such as a sink or a toilet bow. To ensure proper orientation of the discharge outlet above an opening of a waste receptor, the mounting assembly provides for a rigid securing clamp that engages the discharge nozzle and hooks to a rim, or under a rim of the waste receptor.

US 4,722,556 describes a sewerline adapter for a recreational vehicle.

US 5,096,233 and US 5,201,553 both describe standpipe adapters for washing machines.

### SUMMARY

The invention is defined in the claims.

In particular, claim 1 defines a dialysis system.

In the dialysis system, a plug device can include a body configured to be inserted into and secured to an opening of a drain, a hole within the body and extending through the body, and a connector configured to be secured to a drain line of a dialysis system. The hole is configured to receive waste fluid from the drain line of the dialysis system when the connector is secured to the drain line.

The dialysis system can be used in a method (not forming part of the claimed invention) that includes inserting a plug device into a drain, connecting a drain line of a dialysis system to the plug device, and initiating a dialysis treatment in which a dialysis machine of the dialysis system directs waste fluid through the drain line, through the plug device, and into the drain.

Implementations of the foregoing can include one or more of the features described below and herein elsewhere.

Advantages of the foregoing may include, but are not limited to, those described below and herein elsewhere. During PD treatments, the plug devices and guide devices can be used to reduce the chance that waste fluid produced during the dialysis treatments is inadvertently leaked outside of a drain receptacle, e.g., elsewhere into a room where a dialysis treatment is performed or onto a floor surface of the room. By being directly connected to ends of waste fluid lines, the plug devices allow the waste fluid to be pumped directly into the drain, thus simplifying the procedure of disposing of the waste fluid. The plug devices can reduce the need for cleanup of the drain receptacle after dialysis treatments are performed because the waste fluid is disposed of directly into the drain, rather than into a receptacle surrounding the drain. The plug devices can also be engaged to connectors typically used to connect fluid lines of a dialysis system such that specialized connectors need not be used. The plug devices can also be easily secured to drains found in residential and commercial settings.

Rather than directly securing the waste fluid lines to the drains, the guide devices can be used to secure waste fluid lines above the drains. The guide devices can be secured to rim portions of drain receptacles defining the drains so that the guide devices need not be configured to be attached to specific sized drains. The guide devices can be easily secured to rims of drain fixtures found in residential and commercial settings.

The details of one or more implementations of the subject matter described in this specification are set forth in the accompanying drawings and the description below. Other potential features, aspects, and advantages will become apparent from the description, the drawings, and the claims.

### BRIEF DESCRIPTION OF THE DRAWINGS

FIG. 1 is a front perspective view of a dialysis system including a plug device secured to a drain.
FIG. 2 is front perspective view of a dialysis machine of the dialysis system of FIG. 1.
FIGS. 3A and 3B are top perspective and front cross-sectional views, respectively, of an example of a plug device.
FIGS. 4A and 4B are top perspective and front cross-sectional views, respectively, of another example of a plug device.
FIG. 5 is a front perspective view of a dialysis system including a guide device secured to a receptacle portion of a drain fixture.
FIGS. 6A and 6B are front perspective and side views, respectively, of a guide device.
FIG. 7 is a diagram of another example of a dialysis system including a cartridge.
FIG. 8 is a perspective view of a pumping element of the dialysis system of FIG. 7.
FIG. 9 is a perspective view of a dialysis machine of the dialysis system of FIG. 7 without a cartridge inserted into the dialysis machine.
FIG. 10 is a perspective view of the dialysis machine of FIG. 9 with a cartridge inserted into an insertion slot in an open position.
FIG. 11 is a perspective view of the dialysis machine of FIG. 10 with the insertion slot in the closed position.

### DETAILED DESCRIPTION

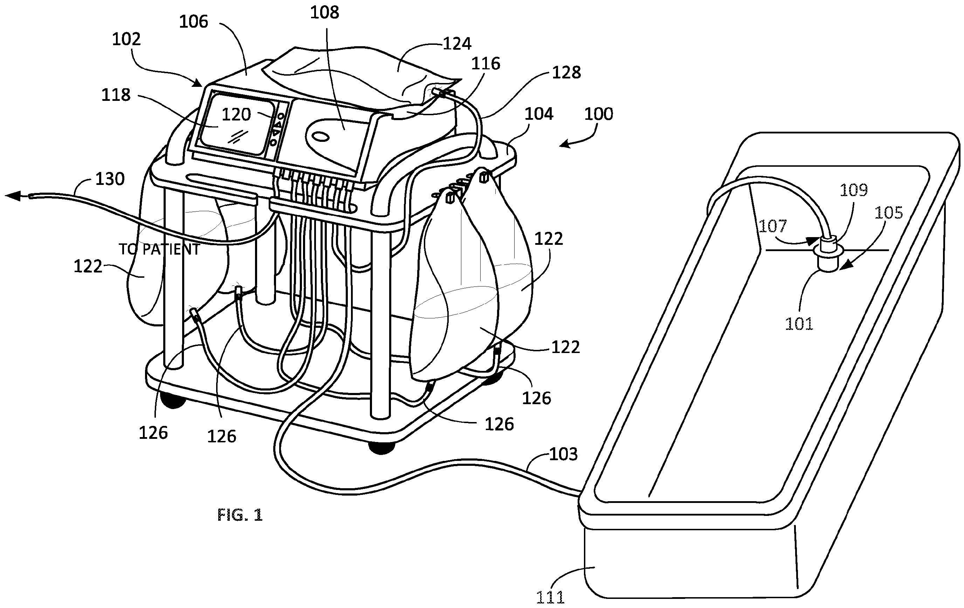

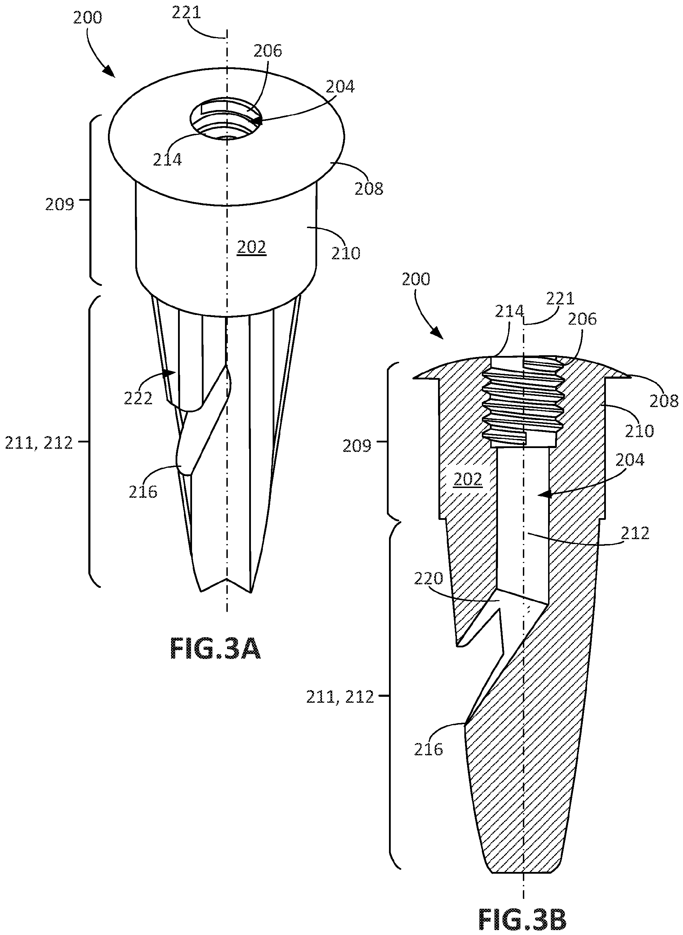

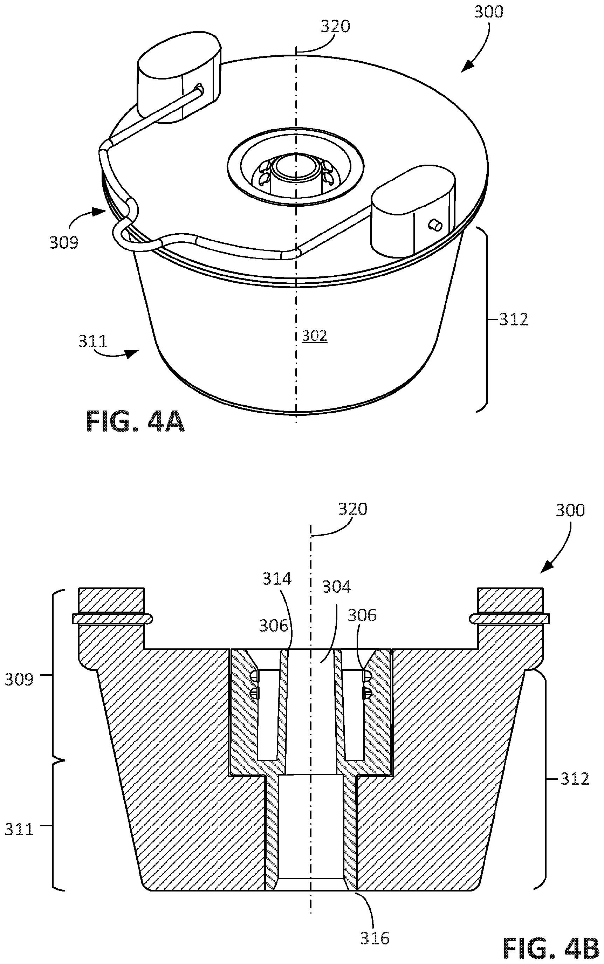

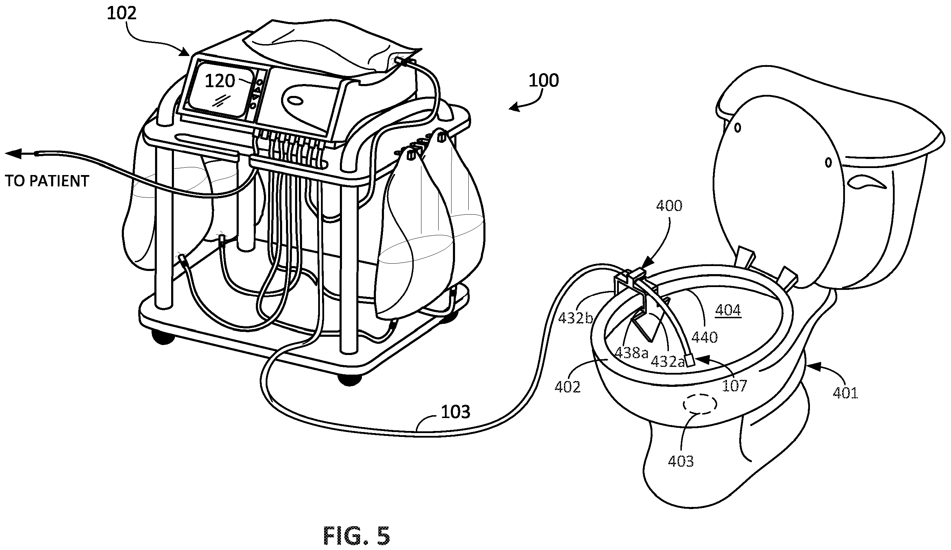

Referring to FIG. 1, a dialysis system 100 can be operated to transfer fluid between a patient and a dialysis machine 102 to move waste products and excess fluids out of the patient's bloodstream into the dialysate solution. The dialysis machine 102 can direct waste fluid containing the waste products through a drain line 103 into a drain fixture 111. In particular, the waste fluid can be directed into a drain 105 of the drain fixture 111, e.g., into an opening 101 of the drain 105. The present disclosure features implementations of systems, methods, and devices that secure the drain line 103 in a manner that can ensure that the waste fluid directed through the drain line 103 is directed into the drain 105. As described herein, in certain implementations, plug devices, such as a plug device 200 shown in FIG. 3 or a plug device 300 shown in FIG. 4, can be used to secure the drain line 103 to the drain 105, whereas in other implementations, guide devices such as a guide device 400 shown in FIG. 5 can be used to guide the drain line 103 to a position over the drain 105.

### Example Dialysis Systems

The dialysis system 100 of FIG. 1 is a peritoneal dialysis system, and the dialysis machine 102 is a peritoneal dialysis machine. The dialysis system 100 includes multiple fluid lines, including the drain line 103, dialysate bag lines 126, a heater bag line 128, and a patient line 130.

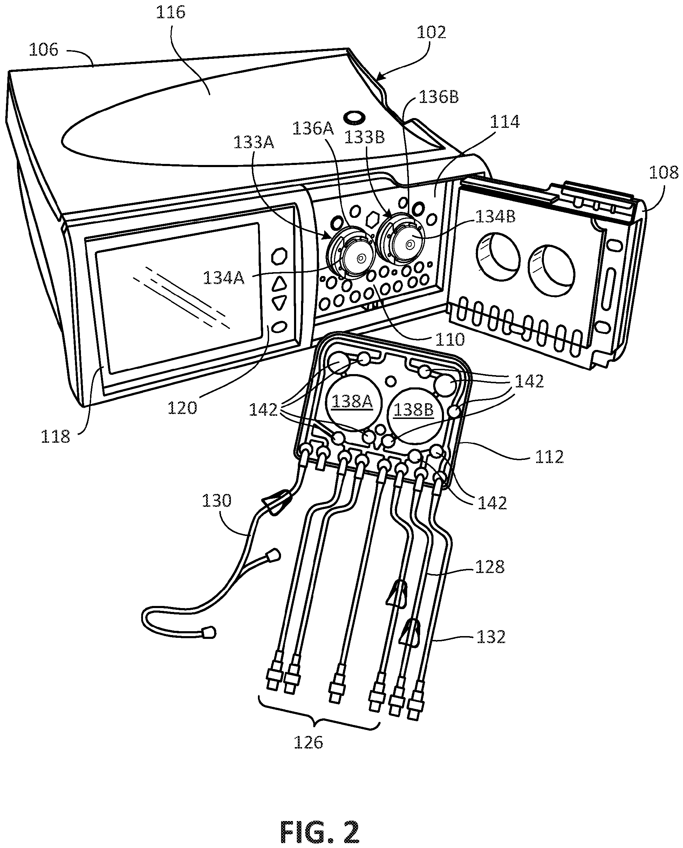

The dialysis machine 102 includes a pumping system (shown in FIG. 2) operable to draw fluid or pump fluid through the various fluid lines of the dialysis system 100. The dialysis machine 102 is seated on a cart 104. Referring also to FIG. 2, the dialysis machine 102 includes a housing 106, a door 108, and a cassette interface 110 that contacts a disposable PD cassette 112 when the cassette 112 is disposed within a cassette compartment 114 formed between the cassette interface 110 and the closed door 108. A heater tray 116 is positioned on top of the housing 106. The heater tray 116 is sized and shaped to accommodate a bag of dialysate (e.g., a 5-liter bag of dialysate). The dialysis machine 102 also includes a touch screen 118 and additional control buttons 120 that can be operated by a user (e.g., a patient or a medical practitioner) to allow, for example, set-up, initiation, and/or termination of a dialysis treatment.

Dialysate bags 122 are suspended from fingers on the sides of the cart 104. The dialysate bags 122 contain fresh dialysate that draw the waste products from the patient's during the dialysate treatment. For example, if the dialysate machine 102 is a PD machine, the waste products are drawn from the patient's blood vessels located in the peritoneal membrane into the dialysate. A heater bag 124 is positioned in the heater tray 116. The dialysate bags 122 and the heater bag 124 are connected to the cassette 112 via the dialysate bag lines 126 and the heater bag line 128, respectively. The dialysate bag lines 126 can be used to pass dialysate from dialysate bags 122 to the cassette 112 during use, and the heater bag line 128 can be used to pass dialysate back and forth between the cassette 112 and the heater bag 124 during use. The patient line 130 and the drain line 103 are also connected to the cassette 112. In implementations in which the dialysis machine 102 is a peritoneal dialysis machine, the patient line 130 can be connected to a patient's abdomen via a catheter and can be used to pass dialysate back and forth between the cassette 112 and the patient's peritoneal cavity during use. An end 107 of the drain line 103 can be directed toward or connected to the drain 105 and can be used to pass dialysate from the cassette 112 to the drain 105.

The dialysis machine 102 includes pistons 133A, 133B with piston heads 134A, 134B that are controlled to move axially inward and outward such that the piston heads 134A, 134B move axially inward and outward within piston access ports 136A, 136B. The pistons 133A, 133B are operated to pump fluid and draw fluid through the fluid lines of the dialysis system 100. For example, the dialysis machine 102 can include stepper motors operable to drive lead screws, which move nuts inward and outward along the lead screws. The nuts, in turn, are connected to the pistons 133A, 133B and thus cause the pistons 133A, 133B to move inward and outward as the stepper motors rotate the lead screws. Stepper motor controllers provide the necessary current to be driven through the windings of the stepper motors to move the pistons 133A, 133B. The polarity of the current determines whether the pistons 133A, 133B are advanced or retracted.

When the cassette 112 is positioned within the cassette compartment 114 of the dialysis machine 102 with the door 108 closed, the piston heads 134A, 134B of the dialysis machine 102 align with pump chambers 138A, 138B of the cassette 112 such that the piston heads 134A, 134B can be mechanically connected to the cassette 112 and engaged with the pump chambers 138A, 138B. In some implementations, once the door 108 is lightly shut and latched, the machine 102 pumps air to force the door 108 against the cassette 112 to create seals between the cassette 112 and the machine 102 so as to prevent fluid leaks from the cassette 112. A vacuum pressure of at least about 2.8 MPa (400 lb./sq. in.) is used, preferably at least 5.5 MPa (800 lb./sq. in.) or more can be used, but 2.8 MPa (400 lb./sq. in.) is usually sufficient.

As a result of this arrangement, movement of the piston heads 134A, 134B toward the cassette 112 during treatment can decrease the volume of the pump chambers 138A, 138B and force dialysate out of the pump chambers 138A, 138B, while retraction of the piston heads 134A, 134B away from the cassette 112 can increase the volume of the pump chambers 138A, 138B and cause dialysate to be drawn into the pump chambers 138A, 138B.

### Example Plug Devices and Related Methods

FIGS. 3A and 3B and FIGS. 4A and 4B depict examples of plug devices 200, 300 that can be used to secure the drain line 103 to the drain 105 (shown in FIG. 1). The plug devices 200, 300 are each configured to be inserted into and secured to the opening 101 of the drain 105. The plug devices 200, 300 are connectable to the drain line 103, e.g., the end 107 of the drain line 103 shown in FIG. 1, such that the waste fluid pumped by the dialysis machine 102 is directed through the end 107 of the drain line 103 and then through the plug device, e.g., the plug device 200 or the plug device 300. The waste fluid, after travelling through the plug device, is directed into the drain 105. The plug devices 200, 300 can ensure that the drain line 103 does not move relative to the drain 105 during a treatment. For example, in the event that a patient undergoing the treatment moves during the treatment and inadvertently jostles the drain line 103 or otherwise causes the drain line 103 to move, the plug devices 200, 300 can ensure that the drain line 103 remains fluidly connected to the drain. Securely coupling the drain line 103 to the drain 105 in this manner can ensure that the waste fluid does not leak to locations outside of the drain 105.

Referring to FIGS. 3A and 3B, the plug device 200 includes a body 202, a hole 204, and a connector 206. The body 202 is an elongate member configured to be inserted into and secured to the opening 101 of the drain 105. In some implementations, the body 202 has a height between 4 cm and 10 cm, and a maximum diameter between 2 cm and 4 cm.

The body 202 is shaped to cover the opening 101 of the drain 105 when the body 202 is inserted into the opening 101 of the drain 105. For example, a flange 208 on a top portion 209 of the body 202 covers the opening 101 of the drain 105 and a cylindrical portion 210 on the top portion 209 of the body 202 contacts a wall of the drain 105 when the plug device 200 is inserted into the opening 101. The cylindrical portion 210 and the wall of the drain 105 form a seal that prevents ingress and egress of fluid. In this regard, fluid can only be dispensed into the drain 105 through the hole 204 and fluid cannot flow from within the drain 105, past the plug device 200, outside of the drain 105, e.g., through backflow. A tapered portion 212 of the body 202 enables the body 202 to be easily inserted into the opening 101 of the drain 105.

The hole 204 extends from a top portion 209 of the body 202, through the body 202, and through a bottom portion 211 of the body 202. The hole 204 provides a path for waste fluid dispensed through the drain line 103 to enter the plug device 200 through a first opening 214 of the hole 204 and exit the plug device 200 through a second opening 216 of the hole 204. The first opening 214 is positioned on the top portion 209 of the body 202, and the second opening 216 is positioned on the bottom portion 211 of the body 202. The hole 204 includes a first portion 218 that extends along a central longitudinal axis 221 of the plug device 200 and a second portion 220 connected to the first portion 218 that is angled relative to the central longitudinal axis 221. The second portion 220 of the hole 204 extends away from the central longitudinal axis 221 of the plug device 200 such that the second portion 220 of the hole 204 and the first portion 218 of the hole 204 are non-parallel. In particular, the second portion 220 of the hole 204 extends at least partially laterally from the first portion 218 of the hole 204 such that the second opening 216 is positioned along a lateral portion 222 of the body 202. In some implementations, an angle between the first portion 218 of the hole 204 and the second portion 220 of the hole 204 is between 30 degrees and 75 degrees (e.g., between 30 degrees and 50 degrees, 40 degrees and 60 degrees, or 50 degrees and 75 degrees).

The connector 206 is positioned along the top portion 209 of the body 202. In particular, the connector 206 is positioned within the first opening 214 of the hole 204 and is configured to receive a corresponding connector 109 on the end 107 of the drain line 103. In some implementations, the connector 206 includes a Luer type fitting configured to connect to a corresponding Luer type fitting on the end 107 of the drain line 103. For example, the Luer type fitting for the connector 206 can be a female connector configured to connect to a male connector on the end 107 of the drain line 103. The connector 206 can include threads that engage with corresponding threads of the connector on the end 107 of the drain line 103. In other implementations, the Luer type fitting for the connector 206 can be a male connector that protrudes outwardly from the body 202 of the plug device 200 and from the first opening 214 of the hole 204. The male connector is configured to connect to a corresponding female connector on the end 107 of the drain line 103.

While FIGS. 1, 3A, and 3B depict one example of a plug device that can be used to secure the drain line 103 to the drain 105, other implementations may feature other types of plug devices. FIGS. 4A and 4B depict another example of a plug device, e.g., the plug device 300. Like the plug device 200, the plug device 300 includes a body 302, a hole 304, and a connector 306. Like the body 202, the body 302 of the plug device 300 is configured to be inserted into and secured to the opening 101 of the drain 105. In some implementations, the body 302 has a height between 1 cm and 6 cm, and a maximum diameter between 3 cm and 8 cm.

The body 302 is shaped to cover the opening 101 of the drain 105 when the body 302 is inserted into the opening 101 of the drain 105. Unlike the body 202, the body 302 does not include a cylindrical portion that forms the seal with the wall defining the drain 105. Rather, the body 302 includes a tapered portion 312 extending through 80% to 95% of an overall height of the body 302, and the tapered portion 312 is pressed against the wall of the drain 105 to create the seal when the body 302 is inserted into the opening 101 of the drain 105. The portion 312, in other implementations, is cylindrical.

Like the hole 204, the hole 304 extends from a top portion 309 of the body 302, through the body 302, and through a bottom portion 311 of the body 302. The hole 304 provides a path for waste fluid dispensed through the drain line 103 to enter the plug device 300 through a first opening 314 of the hole 304 and exit the plug device 300 through a second opening 316 of the hole 304. The first opening 314 is positioned on the top portion 309 of the body 302, and the second opening 316 is positioned on the bottom portion 311 of the body 302. Unlike the hole 204, an entirety of the hole 304 extends along a central longitudinal axis 320 of the plug device 300. As a result, both the first opening 314 and the second opening 316 of the hole 304 are positioned along the central longitudinal axis 320.

The connector 306 is positioned along the top portion 309 of the body 302. The connector 306 is positioned within the first opening 314 of the hole 304 and is configured to receive a corresponding connector on the end 107 of the drain line 103. In some implementations, the connector 306 includes a Luer type fitting configured to connect to a corresponding Luer type fitting on the end 107 of the drain line 103. For example, the Luer type fitting for the connector 306 can be a female connector configured to connect to a male connector on the end 107 of the drain line 103. The connector 306 can include threads that engage with corresponding threads of the connector on the end 107 of the drain line 103. In other implementations, the Luer type fitting for the connector 306 can be a male connector that protrudes outwardly from the body 302 of the plug device 300 and from the first opening 314 of the hole 304. The male connector is configured to connect to a corresponding female connector on the end 107 of the drain line 103.

Example methods of using the dialysis system 100 are described below and herein elsewhere. Methods of operating the dialysis system 100 can include setup, priming, and treatment steps. To setup the dialysis system 100 for treatment, the cassette 112 with the lines 126, 128, 130, 103 attached thereto is positioned in the cassette compartment 114 of the dialysis machine 102 and an inflatable pad in the door 108 of the dialysis machine 102 is inflated such that the cassette 112 is pressed tightly against the cassette interface 110 of the dialysis machine 102. In addition, the end 107 of the drain line 103 is connected to a plug device, e.g., the plug device 200 or the plug device 300, which in turn is secured to the drain 105. When the plug device is inserted into the drain 105, the plug device and the drain 105 form a seal therebetween.

The plug device is connected to the end 107 of the drain line 103 before or after the plug device is inserted into the drain 105. If the plug device includes a Luer fitting, the Luer fitting of the plug device is connected to the corresponding Luer fitting on the end 107 of the drain line 103 to connect the plug device to the end 107 of the drain line 103.

With the cassette 112 properly installed within the cassette compartment 114 of the dialysis machine 102 and the appropriate line connections made, the piston 133A is advanced to initiate the process of mechanically connecting the piston head 134A of the dialysis machine 102 to the cassette 112. After mechanically coupling the piston head 134A of the dialysis machine 102 to the cassette 112, a priming technique is carried out to remove air from the cassette 112 and from the various lines 126, 128, 130, 103 connected to the cassette 112. To prime the cassette 112 and the lines 126, 128, 130, 103, the piston 133A and valves 142 are typically operated to pump dialysate from the heater bag 124 to the drain 105 and from each of the dialysate bags 122 to the drain 105.

A dialysis treatment is then initiated. After priming is complete, the patient line 130 is connected to the patient and the dialysis machine 102 is operated to drain any spent dialysate that was left in the patient's peritoneal cavity from a previous treatment. To drain the spent dialysate from the patient's peritoneal cavity, the valves 142 of the dialysis machine 102 are configured to create an open fluid flow path between the patient line 130 and the pump chamber 138A, and the piston 133A is retracted to draw spent dialysate from the peritoneal cavity of the patient into the pump chamber 138A via the patient line 130. As a result, the volume of the pump chamber 138A is increased and spent dialysate is drawn into the pump chamber 138A from the peritoneal cavity of the patient.

After drawing the dialysate into the pump chamber 138A from the peritoneal cavity of the patient, the valves 142 are configured to create an open fluid flow path between the pump chamber 138A and the drain line 103, and the dialysate is forced out of the pump chamber 138A to the drain 105 by advancing the piston 133A and decreasing the volume of the pump chamber 138A. The dialysis machine 102 directs waste fluid through the drain line 103, through the plug device, and then into the drain 105.

During the patient drain phase of the treatment, the pistons 133A, 133B are typically alternately operated such that the piston 133A is retracted to draw spent dialysate solution into the pump chamber 138A from the patient while the piston 133B is advanced to pump spent dialysate solution from the pump chamber 138B to the drain 105 and vice versa.

To begin the patient fill phase, the valves 142 are configured to create a clear fluid flow path between the pump chamber 138A and the heater bag line 128, and then the piston 133A is retracted to draw warm dialysate from the heater bag 124 to the pump chamber 138A. The warm dialysate travels from the heater bag 124 through the heater bag line 128 and into the pump chamber 138A. The warm dialysate is then delivered to the peritoneal cavity of the patient via the patient line 130 by configuring the valves 142 to create a clear fluid flow path between the pump chamber 138A and the patient line 130 and advancing the piston 133A to pump the warm dialysate into the peritoneal cavity of the patient. During the patient fill phase of the treatment, the pistons 133A, 133B are typically alternately operated such that the piston 133A is retracted to draw warm dialysate into the pump chamber 138A from the heater bag 124 while the piston 133B is advanced to pump warm dialysate from the pump chamber 138B to the patient and vice versa. When the desired volume of dialysate has been pumped to the patient, the dialysis machine 102 transitions from the patient fill phase to a dwell phase during which the dialysate is allowed to sit within the peritoneal cavity of the patient for a long period of time.

During the dwell period, toxins cross the peritoneum of the patient into the dialysate from the patient's blood. As the dialysate dwells within the patient, the dialysis machine 102 prepares fresh dialysate for delivery to the patient in a subsequent cycle. The dialysis machine 102 pumps fresh dialysate from one of the four full dialysate bags 122 into the heater bag 124 for heating. To do this, the pump of the dialysis machine 102 is activated to cause the pistons 133A, 133B to reciprocate and certain valves 142 of the dialysis machine 102 are actuated to cause the dialysate to be drawn into the fluid pump chambers 138A, 138B of the cassette 112 from the selected dialysate bag 122 via its associated line 126. The dialysate is then pumped from the fluid pump chambers 138A, 138B to the heater bag 124 via the heater bag line 128.

After the dialysate has dwelled within the patient for the desired period of time, the spent dialysate is pumped from the patient through the drain line 103 to the drain 105 in the manner described above. The heated dialysate is then pumped from the heater bag 124 to the patient where it dwells for a desired period of time. These steps are repeated with the dialysate from two of the three remaining dialysate bags 122. The dialysate from the last dialysate bag 122 is typically delivered to the patient and left in the patient until the subsequent PD treatment.

After completion of the PD treatment, the pistons 133A, 133B are retracted in a manner to disconnect the piston heads 134A, 134B from the cassette. The door 108 of the dialysis machine 102 is then opened and the cassette 112 is removed from the cassette compartment 114 and discarded.

### Example Guide Devices and Related Methods

The plug devices 200, 300 are examples of devices for securing the end 107 of the drain line 103 to the drain 105. In other implementations, rather than securing the end 107 of the drain line 103 to the drain 105, a device is used to maintain a position or an orientation of the drain line 103 relative to the drain 105, or relative to a drain fixture defining a drain. Referring briefly to FIG. 5, the guide device 400 is secured to a rim portion 402 of a drain fixture 401 defining a drain 403, and the drain line 103 is secured to the guide device 400. In the illustrated embodiment, the drain fixture 401 is a toilet. The guide device 400 can maintain a position of the end 107 of the drain line 103 over a receptacle portion 404 of the drain fixture 401 such that any fluid ejected from the end 107 of the drain line 103 is ejected toward the drain 403.

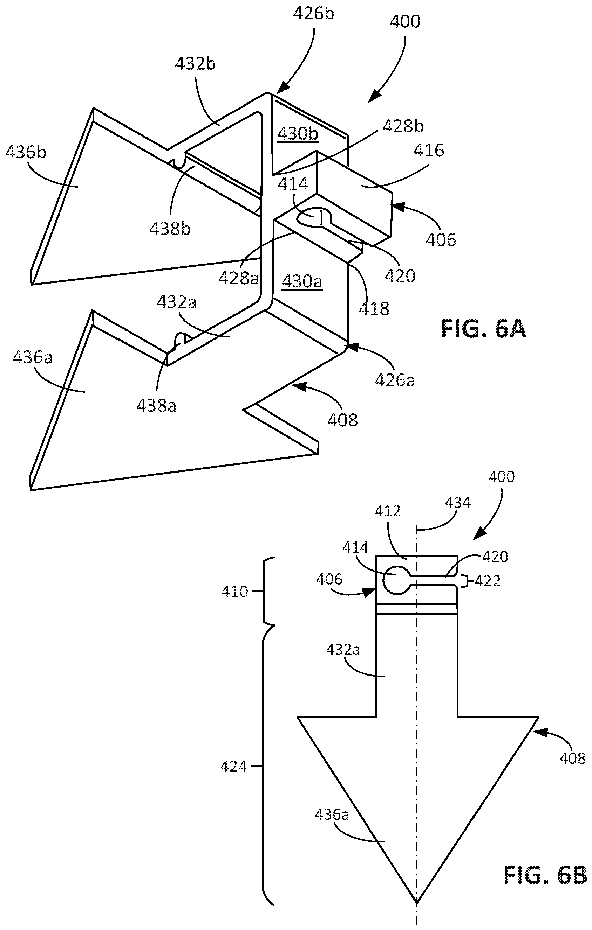

Referring to FIG. 6A, the guide device 400 includes a fluid line guide 406 and a securing mechanism 408. The fluid line guide 406 is on an upper portion 410 of the guide device 400 and is configured to engage the drain line 103 (shown in FIG. 5) and maintain an end of the drain line 103 disposed over the drain 403. The fluid line guide 406 includes a body portion 412 and hole 414 extending laterally through the body portion 412. The body portion 412 includes an upper jaw 416 and a lower jaw 418. The upper jaw 416 and the lower jaw 418 together define the hole 414 and a slot 420.

Referring to FIG. 6B, a height 422 of the slot 420 is less than a diameter of the hole 414. The slot 420 is sized to receive the portion of the drain line 103 after the portion of the drain line 103 is at least partially compressed. The hole 414 is configured to receive the portion of the drain line 103 from the slot 420 and is sized such that the drain line 103 can expand to its normal size after passing through the slot 420. In particular, the hole 414 is large enough such that the drain line 103 can expand to its normal size but small enough such that the connector 109 on the end 107 of the drain line 103 cannot pass through the hole 414.

The diameter of the hole 414 can be larger than a diameter of the drain line 103, e.g., 1.3 mm to 13 mm (0.05 inches to .5 inches) larger than the diameter of the drain line 103. In some examples, the diameter of the hole 414 has a diameter between 0.25 cm to 1.5 cm, e.g., between 0.25 cm and 1.0 cm, 0.5 cm and 1.25 cm, or 0.75 cm and 1.5 cm. In some examples, the height 419 of the slot 420 is between 0.1 cm and 0.5 cm, e.g., between 0.1 cm and 0.3 cm, 0.2 cm and 0.4 cm, or 0.3 cm and 0.5 cm. In some examples, the height 419 of the slot 420 is 25% to 75% of the size of the diameter of the hole 414, e.g., between 25% and 50%, 40% and 60%, or 50% and 75% of the diameter of the hole 414.

Referring back to FIG. 6A, the securing mechanism 408 is on a lower portion 424 of the fluid guide line 406. The securing mechanism 408 is configured to secure the guide device 400 to the rim portion 402 (shown in FIG. 5) of the drain fixture 401. The securing mechanism 408 can include one or more securing members extending downward from the fluid line guide 406, e.g., securing members 426a, 426b. The securing members 426a, 426b are positioned on opposite sides of the fluid line guide 406. For example, the securing member 426a is on a first side 428a of the fluid line guide 406, and the securing member 426b is on a second side 428b of the fluid line guide 406.

The securing members 426a, 426b are symmetric about a central longitudinal axis 434 of the guide device 400 extending through the fluid line guide 406. The securing members 426a, 426b include horizontally extending portions 430a, 430b and vertically extending portions 432a, 432b. The horizontally extending portions 430a, 430b extend outward from the fluid line guide 406 and are configured to rest on the rim portion 402 of the drain fixture 401 when the guide device 400 is secured to the rim portion 402.

The vertically extending portions 432a, 432b extend downward away from the horizontally extending portions 430a, 430b. When the securing mechanism 408 is secured to the rim portion 402 of the drain fixture 401, one of the vertically extending portions 432a, 432b is positioned within the drain fixture 401 and within the receptacle portion 404 of the drain fixture 401, while the other of the vertically extending portions 432a, 432b is positioned outside of the drain fixture 401 and outside of the receptacle portion 404 of the drain fixture 401. For example, as shown in FIG. 5, the vertically extending portion 432a is positioned within the drain fixture 401, and the vertically extending portion 432b is positioned outside of the drain fixture 401.

In some implementations, the vertically extending portions 432a, 432b are first vertically extending portions 432a, 432b, and the guide device 400 further includes second vertically extending portions 436a, 436b that extend downward from the first vertically extending portions 432a, 432b. The second vertically extending portions 436a, 436b can be triangularly shaped. The second vertically extending portions 436a, 436b can serve as counterbalances that facilitate maintaining engagement between the guide device 400 and the rim portion 402 of the drain fixture 401. The second vertically extending portions 436a, 436b have maximum widths 1.5 to 4 times greater than maximum widths of the first vertically extending portions 432a, 432b.

The securing members 426a, 426b also include abutment members 438a, 438b. When the guide device 400 is secured to the drain fixture 401, the abutment member of the one of the vertically extending portions 432a, 432b positioned within the drain fixture 401, e.g., the abutment member 438a of the vertically extending portion 432a, engages a downward facing surface 440 of the rim portion 402. The engagement between the abutment member 438a and the downward facing surface 440 of the rim portion 402 can prevent the guide device 400 from being inadvertently unsecured from the rim portion 402 and can stabilize the guide device 400 such that it does not tilt back and forth during use.

In some implementations, a height of the securing mechanism 408 is between 18 and 46 cm (7 and 18 inches), e.g., between 18 and 33 cm (7 and 13 inches), 25 and 41 cm (10 and 16 inches), or 30 and 46 cm (12 and 18 inches). A height the fluid line guide 406 can be between 0.7 cm and 2.7 cm, e.g., between 0.7 cm and 1.5 cm, 1.2 cm and 2.0 cm, or 1.7 cm and 2.7 cm. A height of the securing mechanism can be between 70% and 95% of an overall height of the guide device 400, e.g., between 75% and 95%, 80% and 95%, or 85% and 95% of the overall height of the guide device 400.

Methods of operating the dialysis system 100 that include use of the guide device 400 in the manner shown in FIG. 5 are similar to the example methods of using the dialysis system 100 described in connection with FIG. 1. The methods of using the guide device 400 differ in that, rather than connecting the end 107 of the drain line 103 to the plug device, a portion of the drain line 103 is secured to the guide device 400. In particular, referring also to FIG. 5, in setting up for treatment, the portion of the drain line 103 is engaged to the guide device 400, and the guide device 400 is secured to the rim of the drain fixture 401.

To engage the portion of the drain line 103 to the guide device 400, the portion of the drain line 103 is partially compressed and inserted into the slot 420. The drain line 103 is then repositioned into the hole 414 and allowed to expand into its normal shape.

To secure the guide device 400 to the rim of the drain fixture 401, the guide device 400 is set on the rim portion 402 of the drain fixture 401 such that the horizontally extending portions 430a, 430b rest on the rim portion 402. In addition, the vertically extending portions 432a extends downward into the receptacle portion 404 and hence into the drain fixture 401, and the vertically extending portion 432b extends downward outside of the drain fixture 401 and hence outside of the receptacle portion 404. The abutting member 438a also engages with the downward facing surface 440.

The portion of the drain line 103 is engaged to the guide device 400 such that the end 107 of the drain line 103 is disposed over the receptacle portion 404 of the drain fixture 401. In this regard, waste fluid directed through the drain line 103 is directed into the receptacle portion 404 rather than directly into the drain 403 as is the case for methods using the plug device. The end 107 of the drain line 103 is left freely hanging in the receptacle portion 404 such that the waste fluid is flown into the receptacle portion 404 and then into the drain 403 during treatment.

### Other Alternative Implementations

A number of implementations have been described. Nevertheless, it will be understood that various modifications may be made.

The system 100 and the machine 102 have been described as a dialysis system and a dialysis machine, respectively. In other implementations, the system and the machine are part of extracorporeal medical fluid treatment systems that can perform operations such as blood filtration, hemofiltration, blood donation, blood detoxification, apheresis, cardiac catheterizations, among other blood treatment procedures. In some implementations, rather than being peritoneal dialysis systems and machines, the dialysis system 100 and the dialysis machine 102 are a hemodialysis system and a hemodialysis machine, respectively, that can perform a hemodialysis treatment.

In some implementations, the machine 102 includes an online dialysis generation system that produces dialysate using a standard household, commercial, or industrial water source. The dialysis generation system produces the dialysate by filtering water from the water source.

In some implementations, the machine 102 includes an online heater that heats dialysate before the dialysate is delivered to the patient.

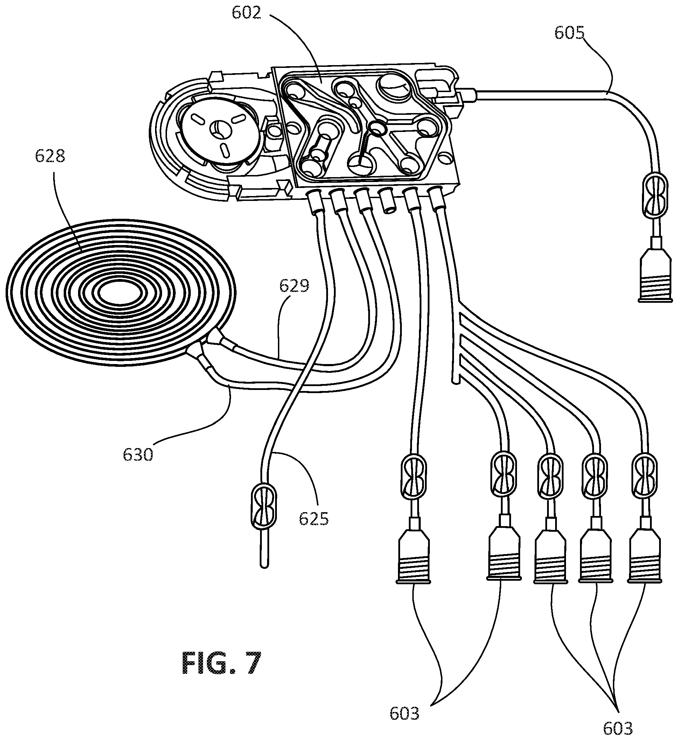

In some implementations, rather than relying on piston pumps, a dialysis system includes a peristaltic pump that pumps fluid through fluid lines of the dialysis system. FIGS. 7-10 depict a dialysis system 600 including a pumping element 601, a cartridge 602, and a dialysis 006Dachine 651, e.g., a peritoneal dialysis cycler. The dialysis system 600 is similar to the dialysis system 100 except that the dialysis system 600 uses a peristaltic pump to pump fluid to and from the patient, the drain, or other destinations.

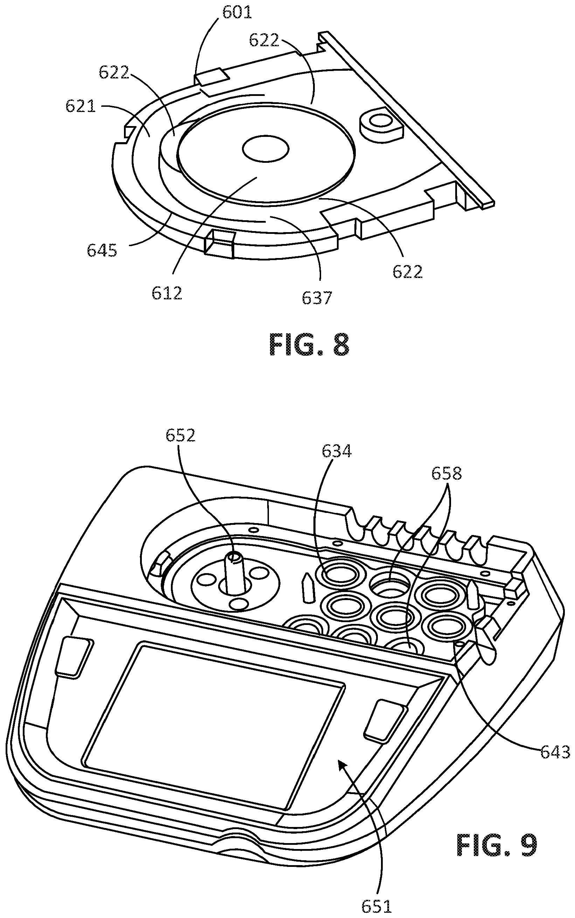

FIG. 7 shows an assembly including the pumping element 601 and the cartridge 602. The pumping element 601 and the cartridge 602, in some implementations, are separate devices that are attachable to one another and each removable from the dialysis machine 651. In other implementations, the cartridge 602 is fixed to the pumping element 601. The cartridge 602 is also fixed to a patient line 605, supply bags 603, a warmer enter line 629, a warmer outer line 630 and a warmer pouch 628. The patient line 605 is connected to the patient during a dialysis treatment performed by the dialysis machine 651 (shown in FIG. 8). The supply bags 603 provide a source of dialysate for the dialysis machine 651. The warmer pouch 628 is essentially made of a fluid circuit within a plastic bag (e.g. a PVC bag) to be put into contact with a warming plate (not shown) of the dialysis system 600. The warming plate is part of a warming system of the dialysis system 600 that provides heat to the warming pouch 628. The warming pouch 628 is composed of a liquid channel which forces the liquid to be maintained within the warming system for a certain duration at a given flow rate.

The pumping element 601 is a peristaltic pump. As shown in FIG. 8, the pumping element 601 includes a pump casing 645 containing three rollers 622 maintained around a center of the pump casing 645 by a roller separator 612. The space between the roller separator 612 and the pump casing 645 defines a pump race 621 in which a flexible tube 637 is placed. The flexible tube 637 is connected with the pump enter 656 and exit 657 lines. The rollers 622 may be motor driven by a shaft 652 of the dialysis machine 651 (shown in FIG. 9) in such a way as to progressively compress the flexible tube 637 resulting thereby in a peristaltic movement along the flexible tube 637.

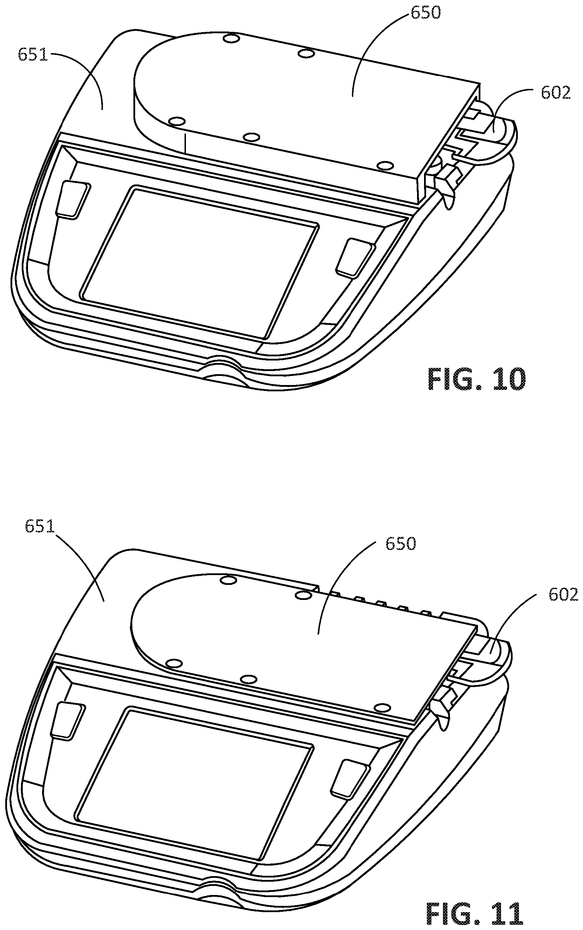

FIG. 9 shows the dialysis machine 651 without the cartridge 602 and the pumping element 601. It contains a driving zone that includes the motor shaft 652 for engaging with the rollers 622 and several actuators 634 operable to actuate valves on the cartridge 602. The dialysis machine 651 also includes an air sensor 643 situated close to the patient line 605 when the cartridge 602 is inserted. The air sensor 643 may be made of a piezo emitter and a piezo receiver. FIG. 10 shows the dialysis machine 651 of FIG. 9 containing the cartridge 602.

To set up for treatment, the cartridge 602 is connected to the machine 651 by sliding the cartridge 602 into a drawer of the machine 651, e.g., into an insertion slot 650 as shown in FIG. 10. The insertion slot 650 is in an open position in FIG. 10. As shown in FIG. 11, the insertion slot 650 is then placed in a closed position. In particular, the cartridge 602 is then lowered to engage the pumping unit 601, in particular to engage the rollers 622 and the valves of the cartridge 602. When the cartridge 602 is connected to the dialysis machine 651. The dialysis machine 651 can then drive the rollers 622 and the valves of the cartridge 602 to move fluid through the fluid lines of the dialysis machine 651.

As shown in FIG. 1, the drain fixture 111 is depicted as a bathtub, and, as shown in FIG. 5, the drain fixture 401 is depicted as a toilet. In other implementations, the drain fixtures 111, 401 correspond to bathroom sinks, laundry room sinks, kitchen sinks, shower drain fixtures, or other appropriate drain fixtures. The receptacle portion 404, as shown in FIG. 5, can correspond to a toilet bowl. In other implementations, the receptacle portion 404 corresponds to a receptacle portion of a bathtub or a sink.

While the plug device 200 has been described as including a cylindrical portion 210 that contacts the wall of the drain 105 to form a seal, in some implementations, the plug device 200 includes an additional component or portion positioned along the cylindrical portion 210 to form the seal. For example, the plug device 200 can further include a gasket or an elastomeric portion that contacts the wall of the drain 105 to form the seal. The gasket or the elastomeric portion is compressed between the cylindrical portion 210 and the wall of the drain 105 to form the seal.

Accordingly, other implementations are within the scope of the claims.

## Claims

1. A dialysis system comprising:
a dialysis machine (102);
a drain line (103) configured to be connected to the dialysis machine to direct waste fluid from the dialysis machine to a drain fixture (111) having a drain (105);
**characterized in that** the dialysis system comprises further
a plug device (200, 300) configured to be inserted into and secured to an opening (101) of the drain (105) of the drain fixture (111), wherein the plug device is connectable to the drain line to allow the waste fluid to be directed from the dialysis machine, through the drain line, through the plug device, and directly into the drain, rather than into the drain fixture.

2. The dialysis system of claim 1, wherein the plug device comprises:
a body (202, 302) configured to be inserted into and secured to the opening of the drain,
a hole (204, 304) extending through the body; and
a connector (206, 306) in the opening, the connector configured to be secured to the drain line of the dialysis system.

3. The dialysis system of claim 2, wherein the connector comprises a Luer fitting, wherein the Luer fitting is configured to receive a corresponding Luer fitting of the drain line.

4. The dialysis system of any of claims 2-3, wherein at least a portion of the hole extends along a central axis (221, 320) of the plug device.

5. The dialysis system of any of claims 2-4, wherein an entire length of the hole extends along a central axis of the plug device.

6. The dialysis system of any of claims 2-5, wherein the hole extends from a top portion (209, 309) of the body, through the body to a lateral portion of the body.

7. The dialysis system of any of claims 2-6, wherein the hole comprises a first portion (218) connected to a second portion (220), the first portion extending along a central axis of the plug device, and the second portion extending away from the central axis such that the second portion and the first portion are non-parallel to one another.

8. The dialysis system of any of claims 1-7, wherein the drain fixture (111) is a bathtub and the drain is a drain of the bathtub.

9. The dialysis system of any of claims 1-7, wherein the drain fixture (111) is a sink and the drain is a drain of the sink.

10. The dialysis system of any of claims 1-9, wherein the plug device is configured to form a seal with a wall defining the opening of the drain.

11. The dialysis system of any of claims 1-10, wherein the dialysis machine comprises a peritoneal dialysis machine.

12. The dialysis system of claim 2, claim 3, claim 4, claim 5, or claim 7 wherein:
the hole is within the body; and
the hole is configured to receive waste fluid from the drain line of the dialysis system when the connector is secured to the drain line.

13. The dialysis system of claim 12, wherein the body is configured to form a seal with a wall defining the opening of the drain.

14. The dialysis system of any of claims 12-13, wherein the hole extends from a top portion of the body, through the body, and to a lateral portion of the body.

15. The dialysis system of any of claims 12-14, wherein the connector is within the hole of the plug device.

16. The dialysis system of any preceding claim, further comprising the drain fixture (111) having the drain (105).

## Patentansprüche

1. Dialysesystem umfassend:
die Dialysemaschine (102),
eine Ablaufleitung (103), die dazu ausgelegt ist, mit der Dialysemaschine verbunden zu werden, um Flüssigabfall von der Dialysemaschine zu einer Ablaufarmatur (111) mit einem Ablauf (105) zu leiten,
**dadurch gekennzeichnet, dass** das Dialysesystem ferner Folgendes umfasst:
eine Stopfenvorrichtung (200, 300), die dazu ausgelegt ist, in eine Öffnung (101) des Ablaufs (105) der Ablaufarmatur (111) eingeführt und daran befestigt zu werden, wobei die Stopfenvorrichtung mit der Ablaufleitung verbindbar ist, damit der Flüssigabfall von der Dialysemaschine durch die Ablaufleitung, durch die Stopfenvorrichtung und direkt in den Ablauf und nicht in die Ablaufarmatur geleitet werden kann.

2. Dialysesystem nach Anspruch 1, wobei die Stopfenvorrichtung Folgendes umfasst:
einen Körper (202, 302), der dazu ausgelegt ist, in der Öffnung des Ablaufs eingeführt und daran befestigt zu werden,
ein Loch (204, 304), das sich durch den Körper erstreckt, und
einen Verbinder (206, 306) in der Öffnung, wobei der Verbinder dazu ausgelegt ist, an der Ablaufleitung des Dialysesystems befestigt zu werden.

3. Dialysesystem nach Anspruch 2, wobei der Verbinder einen Luer-Anschluss umfasst, wobei der Luer-Anschluss zur Aufnahme eines entsprechenden Luer-Anschlusses der Ablaufleitung ausgelegt ist.

4. Dialysesystem nach einem der Ansprüche 2 - 3, wobei sich mindestens ein Abschnitt des Lochs entlang einer mittleren Achse (221, 320) der Stopfenvorrichtung erstreckt.

5. Dialysesystem nach einem der Ansprüche 2 - 4, wobei sich eine gesamte Länge des Lochs entlang einer mittleren Achse der Stopfenvorrichtung erstreckt.

6. Dialysesystem nach einem der Ansprüche 2 - 5, wobei sich das Loch von einem oberen Abschnitt (209, 309) des Körpers durch den Körper zu einem seitlichen Abschnitt des Körpers erstreckt.

7. Dialysesystem nach einem der Ansprüche 2 - 6, wobei das Loch einen ersten Abschnitt (218) umfasst, der mit einem zweiten Abschnitt (220) verbunden ist, wobei sich der erste Abschnitt entlang einer mittleren Achse der Stopfenvorrichtung erstreckt und sich der zweite Abschnitt von der mittleren Achse weg erstreckt, so dass der zweite Abschnitt und der erste Abschnitt nicht zueinander parallel verlaufen.

8. Dialysesystem nach einem der Ansprüche 1 - 7, wobei die Ablaufarmatur (111) eine Badewanne und der Ablauf ein Ablauf der Badewanne ist.

9. Dialysesystem nach einem der Ansprüche 1 - 7, wobei die Ablaufarmatur (111) ein Ausguss und der Ablauf ein Ablauf des Ausgusses ist.

10. Dialysesystem nach einem der Ansprüche 1 - 9, wobei die Stopfenvorrichtung dazu ausgelegt ist, eine Dichtung mit einer Wand zu bilden, die die Öffnung des Ablaufs definiert.

11. Dialysesystem nach einem der Ansprüche 1 - 10, wobei die Dialysemaschine eine Peritonealdialysemaschine umfasst.

12. Dialysesystem nach Anspruch 2, Anspruch 3, Anspruch 4, Anspruch 5 oder Anspruch 7, wobei:
das Loch in dem Körper ist und
das Loch dazu ausgelegt ist, Flüssigabfall von der Ablaufleitung des Dialysesystems aufzunehmen, wenn der Verbinder an der Ablaufleitung befestigt ist.

13. Dialysesystem nach Anspruch 12, wobei der Körper dazu ausgelegt ist, eine Dichtung mit einer Wand zu bilden, die die Öffnung des Ablaufs definiert.

14. Dialysesystem nach einem der Ansprüche 12 - 13, wobei sich das Loch von einem oberen Abschnitt des Körpers durch den Körper zu einem seitlichen Abschnitt des Körpers erstreckt.

15. Dialysesystem nach einem der Ansprüche 12 - 14, wobei sich der Verbinder in dem Loch der Stopfenvorrichtung befindet.

16. Dialysesystem nach einem der vorhergehenden Ansprüche, ferner umfassend die Ablaufarmatur (111) mit dem Ablauf (105).

## Revendications

1. Système de dialyse comprenant :
une machine de dialyse (102) ;
un conduit de vidange (103) conçu pour être raccordé à la machine de dialyse pour acheminer du fluide de rejet de la machine de dialyse à un dispositif de vidange (111) comportant un orifice de vidange (105) ;
**caractérisé en ce que** le système de dialyse comprend, en outre,
un dispositif de bouchon (200, 300) conçu pour être inséré et fixé dans une ouverture (101) de l'orifice de vidange (105) du dispositif de vidange (111), le dispositif de bouchon étant propre à être raccordé au conduit de vidange pour permettre l'acheminement du fluide de rejet depuis la machine de dialyse, à travers le conduit de vidange, à travers le dispositif de bouchon, et directement dans l'orifice de vidange, plutôt que dans le dispositif de vidange.

2. Système de dialyse selon la revendication 1, dans lequel le dispositif de bouchon comprend :
un corps (202, 302) conçu pour être inséré et fixé dans l'ouverture de l'orifice de vidange,
un trou (204, 304) s'étendant à travers le corps ; et
un raccord (206, 306) dans l'ouverture, le raccord étant conçu pour être fixé à la conduite de vidange du système de dialyse.

3. Système de dialyse selon la revendication 2, dans lequel le raccord comprend un raccord Luer, dans lequel le raccord Luer est conçu pour recevoir un raccord Luer correspondant de la conduite de vidange.

4. Système de dialyse selon l'une ou l'autre des revendications 2 et 3, dans lequel au moins une partie du trou s'étend le long d'un axe central (221, 320) du dispositif de bouchon.

5. Système de dialyse selon l'une quelconque des revendications 2 à 4, dans lequel une longueur entière du trou s'étend le long d'un axe central du dispositif de bouchon.

6. Système de dialyse selon l'une quelconque des revendications 2 à 5, dans lequel le trou s'étend depuis une partie supérieure (209, 309) du corps, à travers le corps, jusqu'à une partie latérale du corps.

7. Système de dialyse selon l'une quelconque des revendications 2 à 6, dans lequel le trou comprend une première partie (218) raccordée à une seconde partie (220), la première partie s'étendant le long d'un axe central du dispositif de bouchon, et la seconde partie s'étendant de façon à s'éloigner de l'axe central de telle sorte que la seconde partie et la première partie ne sont pas parallèles l'une à l'autre.

8. Système de dialyse selon l'une quelconque des revendications 1 à 7, dans lequel le dispositif de vidange (111) est une baignoire et l'orifice de vidange est un orifice de vidange de la baignoire.

9. Système de dialyse selon l'une quelconque des revendications 1 à 7, dans lequel le dispositif de vidange (111) est un évier et l'orifice de vidange est un orifice de vidange de l'évier.

10. Système de dialyse selon l'une quelconque des revendications 1 à 9, dans lequel le dispositif de bouchon est conçu pour établir un contact étanche avec une paroi définissant l'ouverture de l'orifice de vidange.

11. Système de dialyse selon l'une quelconque des revendications 1 à 10, dans lequel la machine de dialyse comprend une machine de dialyse péritonéale.

12. Système de dialyse selon la revendication 2, la revendication 3, la revendication 4, la revendication 5 ou la revendication 7, dans lequel :
le trou est situé à l'intérieur du corps ; et
le trou est conçu pour recevoir du fluide de rejet provenant du conduit de vidange du système de dialyse lorsque le raccord est fixé au conduit de vidange.

13. Système de dialyse selon la revendication 12, dans lequel le corps est conçu pour établir un contact étanche avec une paroi définissant l'ouverture de l'orifice de vidange.

14. Système de dialyse selon l'une ou l'autre des revendications 12 et 13, dans lequel le trou s'étend depuis une partie supérieure du corps, à travers le corps et jusqu'à une partie latérale du corps.

15. Système de dialyse selon l'une quelconque des revendications 12 à 14, dans lequel le raccord est situé à l'intérieur du trou du dispositif de bouchon.

16. Système de dialyse selon l'une quelconque des revendications précédentes, comprenant, en outre, le dispositif de vidange (111) comportant l'orifice de vidange (105).
